Europäisches Patentamt

European Patent Office

Office européen des brevets

(11) Publication number: **0 382 508 B1**

# EUROPEAN PATENT SPECIFICATION

(45) Date of publication of patent specification: **30.11.94**  (51) Int. Cl.⁵: **C07C 209/48**

(21) Application number: **90301290.4**

(22) Date of filing: **07.02.90**

(54) **The preparation of polyamines.**

(30) Priority: **07.02.89 US 306930**
**05.02.90 US 475557**

(43) Date of publication of application:
**16.08.90 Bulletin 90/33**

(45) Publication of the grant of the patent:
**30.11.94 Bulletin 94/48**

(84) Designated Contracting States:
**AT BE CH DE DK ES FR GB GR IT LI LU NL SE**

(56) References cited:
**EP-A- 0 212 986**
**US-A- 3 565 957**

(73) Proprietor: **HAMPSHIRE CHEMICAL CORPO-
RATION**
**55 Hayden Avenue**
**Lexington, Massachusetts 02173 (US)**

(72) Inventor: **O'Neill, Gerald Joseph**
**70 Ridge Street**
**Arlington, Massachusetts 02174 (US)**
Inventor: **Levesque, Albert Henry**
**South Groton Street**
**Nashua, New Hampshire 03060 (US)**

(74) Representative: **Bentham, Stephen et al**
**J.A. KEMP & CO.**
**14 South Square**
**Gray's Inn**
**London WC1R 5LX (GB)**

EP 0 382 508 B1

**Description**

The present invention relates to an improved process for forming non-cyclic, aliphatic compounds having a multiplicity of primary amino groups and forming these compounds in high yields and selectivity from the corresponding polynitrile having from 2 to 3 atoms between the cyano groups.

The hydrogenation of a wide variety of nitriles to their corresponding amines using conventional hydrogenation catalysts is well known. However, it is recognized that this mode of synthesis leaves much to be desired when the aim is to produce non-cyclic, aliphatic polyamines from polynitriles having an atomic structure capable of forming five and six membered ring containing compounds. The presently known processes often provide the desired noncyclic products in low selectivity and yield, while the undesired cyclic compounds predominate. This is especially true with polynitriles such as nitrilotriacetonitrile (NTAN), iminodiacetonitrile (IDAN) and ethylenediaminetetraacetonitrile (EDTN). Normally, the dominant products formed are cyclic polyamines. When one attempts to adjust the reaction conditions to those which may provide higher selectivity or yield of the noncyclic, aliphatic compound, one observes rapid inactivation of the catalyst materials used.

It is generally known that hydrogenation of nitriles can be accomplished by many modes such as by batch processing using a stirred autoclave or by continuous processing using a fixed bed reactor to contact a hydrogenation catalyst with a solution containing a nitrile. The reaction product is generally a mixture of primary, secondary and tertiary amines. The secondary and tertiary amines are by-product materials which are thought to occur by the reaction of some of the primary amine product with imine intermediate material (formed in the hydrogenation of the nitrile). In this process, the already formed primary amine reacts with imine intermediate to produce a secondary amine and, in turn, some of the secondary amine reacts with additional imine to produce a tertiary amine product.

When the starting nitrile has a multiplicity of cyano groups which are separated by an appropriate chain length of about 2 to 3 atoms, the secondary and tertiary amine formation tends to be intramolecular to provide cyclic compounds as the dominant product. Thus, when a dinitrile, such as iminodiacetonitrile, is subjected to conventional hydrogenation, one forms the cyclic compound, piperazine, as the major material. For a trinitrile, such as nitrilotriacetonitrile, the difficulty of forming the corresponding linear aliphatic amine, tris (2-amino-ethyl) amine (TREN), increases geometrically. Thus, contacting of a polynitrile with a hydrogenation catalyst is a recognized route for producing cyclic polyamines.

The use of a batch reactor has been previously viewed as a process mode which promotes the formation of unwanted side products. The batch reactor normally used in this high pressure reaction is, by conventional design, a fixed vessel in which all of the reactants are initially charged into the reaction vessel and all of the primary amine product is retained within the vessel until the process is terminated. As the nitrile is converted to imine intermediate, there is an increased probability for it to react with previously formed amine contained within the vessel and be diverted into by-product formation. Thus, when polynitriles are converted into polyamines using a conventional batch reaction, one conventionally obtains large amounts of cyclic product as well as methylated secondary amine and condensation by-products. U.S.Pat.Nos.3,565,957 and 3,733,325 teach that the yields of the cyclic amine can be optimized by carrying out the reaction in the presence of large amounts of ammonia. By using a hydrogenation catalyst in the absence of ammonia to increase the yield of linear product, one produces solid condensation material which inactivates the catalyst in a very short period of time. Further, U.S.3,565,957 provides an example which attempts to teach the formation of linear amine, TREN, but clearly the product formed therein is not TREN as the product has a B.P. which is distinctly different from TREN. This reference thus exemplifies the difficulty in forming desired linear polyamine product by a batch mode. The short life of the catalyst as well as low selectivity and yield has caused this process to be deemed commercially unfeasible in providing linear polyamines.

To overcome the problems associated with synthesizing aliphatic polyamines from polynitriles in batch reactors, the use of a fixed bed (trickle-bed) reactor has been suggested by Sherwin et al. in U.S. Patent No.4,721,811. While the fixed bed reactor can efficiently produce large quantities of the desired amines, it cannot match the cost-effectiveness of batch-type reactor vessels when relatively smaller output is contemplated. The fixed bed process is relatively complex to set up and generally must be customized for the particular reaction to be carried out. Once set up, it is quite inefficient to modify the fixed bed reactor to accommodate a different process and then re-modify the apparatus for the original process. Thus, the fixed bed reactor process is best suited for those situations where the apparatus can be dedicated to one single reaction process. Additionally, heat removal can be a special problem with such fixed bed reactors, for example in the reduction of nitro and nitrile compounds which have very high heats of hydrogenation.

The present process would enable one to provide an efficient discontinuous or batch process carried out in a batch reactor, e.g., a stirred autoclave reactor vessel, which once the desired quantity of product is produced, is easily and cost effectively capable of being changed over to use in another process.

The present invention provides a process for the batchwise preparation of polyamines comprising hydrogenating a solution of the corresponding polynitrile in which the nitrile groups are separated by from 2 to 3 atoms (and thus have an atomic structure which is capable of forming five and six membered ring containing compounds).

The present process requires the contacting of a polynitrile having its cyano groups separated by from 2 to 3 atoms, with a Raney cobalt catalyst under hydrogen pressure. The polynitrile is introduced into the reaction vessel as a solution over substantially the entire time the process is carried out and at a rate such that the molar concentration of nitrile in the reaction zone is equal to or below 3 percent of the concentration of the feed supply throughout the reaction.

The present invention is directed to a fed-batch process which provides for the preparation of an aliphatic polyamine from its corresponding polynitrile. The present invention is directed to a process which uses a combination of specific parameters to unexpectedly provide a means of forming aliphatic primary amines in high conversion and selectivity from polynitriles, provides extended catalyst activity and permits production of purified material by yielding a product stream which can be readily separated.

The term "fed-batch" reactor described in the present specification and appended claims is a reactor which has a reaction zone in which at least one liquid (e.g., polynitrile solution) reactant is introduced into the reaction zone over substantially the entire time of the batch reaction, the reactants are contained therein and the products remain therein until completion of the batch reaction. A fed-batch reactor is distinct in use and design from a batch reactor (in which the reactants, the charge, are introduced into the reaction zone before the process is begun and the reactants and products are allowed to remain in the reaction zone until completion of the batch reaction, as defined by a preset time, yield or both.

The term "polynitrile" as used herein and in the appended claims defines compounds having at least two cyano groups separated by an immediate chain of 2 or 3 atoms. The cyano groups may be separated by hydrocarbon chains which are saturated or contain olefinic (ethylenic) unsaturation therein or may contain a heteroatom such as nitrogen, oxygen or sulfur, or combinations thereof. Specifically, the most applicable polynitriles are nitrilotriacetonitrile, iminodiacetonitrile and ethylenediaminetetraacetonitrile as these materials provide highly desired noncyclic polyamines in an economical manner. Other polynitriles include oxidiacetonitrile, thiodiacetonitrile, 2-methylglutaronitrile and 1,3-dicyanopropene. These compounds are normally viewed as having the proper atom chain length to intramolecularly react and form stable cyclic compounds as the dominant product. However, the present process provides a means to cause the dominant product to be an aliphatic, noncyclic polyamine.

The term "polyamine" as used herein and in the appended claims refers to compounds having a plurality of amino groups corresponding to the polynitrile structure, above, wherein each nitrile is converted into a primary $-CH_2NH_2$ group.

The process involves contacting, preferably in a stirred or otherwise well agitated autoclave reactor vessel, a polynitrile and catalyst with hydrogen gas under pressure. The catalyst is preferably a finely divided Raney cobalt and, most preferably, is chromium-modified ("chromium-promoted") Raney cobalt. A valuable modification to the process includes conducting the hydrogenation in the presence of ammonia.

The process must be carried out in a fed-batch reactor capable of having a solution of the polynitrile reactant introduced into the reaction zone from the time of commencement of the reaction to a time substantially that of completion of the batch reaction. The solution of polynitrile should be added to the reaction zone at a rate which is not greater than the maximum rate at which the polynitrile within the reaction zone is reacting with hydrogen. It is preferred that the gradual addition of the polynitrile reactant be conducted at a rate such that the molar concentration of polynitrile within the reactor is equal to or less than three (3) mole percent of the polynitrile within the feed solution. This relationship of feed concentration to reaction zone concentration can be readily monitored by conventional means, such as, in which the reaction zone concentration is monitored by gas chromatography and the reactant solution income flow rate is adjusted accordingly.

The gradual addition of the polynitrile reactant should be conducted at a rate such that the space velocity of the polynitrile is within the range of 0.1 to 1.3, preferably from 0.8 to 1.2 and most preferably about 1. When the rate is increased above 1.3, the desired product yield and selectivity drastically diminish and the catalyst activity is shortened so that the catalyst is not suitable for subsequent runs (thus increasing the cost of the process). The space velocity for a particular fed-batch system can be readily calculated by the rate of polynitrile input by weight per hour divided by the weight of catalyst in the fed-batch reactor in use.

3

The volumetric feed rate can also be calculated and properly maintained to achieve the required feed to reaction zone polynitrile concentrations by insuring that a reaction parameter K, which is defined below, is maintained at a numerical value of at least 30 or greater. The parameter K for any particular system can be defined as:

$$K = \frac{163 \ WP}{nV_f} \cdot e^{-\frac{4026}{T+273}}$$

wherein

W = amount of catalyst in the reaction zone in kilograms;

P = hydrogen pressure in the reaction zone in atmospheres;

$V_f$ = volumetric feed rate of the polynitrile feed solution into the reactor in cubic meters per hour;

n = number of nitrile groups per mole of feed; and

T = reaction zone temperature in °C.

The polynitrile must be introduced into the reaction zone as a liquid and, therefore, is normally introduced as a solution in a solvent medium. Solvents suitable for this purpose include amides such as N,N-dimethylacetamide (DMAC), formamide and N,N-dimethylformamide (DMF), ethers, such as dimethoxypropane and dioxan as well as other solvents which are inert to the reactants and the products in the reaction zone and are capable of remaining liquid under the reaction conditions. It is preferred that the polynitrile be introduced as a solution at concentrations of from 5 wt. percent to saturation, preferably from 5 to 30 wt. percent based on the total weight of the liquid solution introduced into the reaction zone. Higher concentrations can be utilized, e.g., up to about 70 wt. percent, when the feed solution is maintained at an elevated temperature. Because the polynitriles are soluble in the commonly employed solvents, a substantially saturated solution of polynitrile is consequently preferred. For example, in the preferred DMAC solvent, it is possible to obtain a fully saturated solution of about 22.5% by weight NTAN at ambient conditions. The employment of a "substantially saturated" solution of about 20% by weight of NTAN in DMAC, or about 25% by weight in DMF, is preferred to avoid the possibility of having the polynitrile come out of solution during the processing. After preparation, the solution is sparged with nitrogen until all air is removed. The feedstock solution is maintained under a nitrogen blanket pending further use.

It is understood that the specific polynitrile reactant chosen will determine the primary polyamine product to be formed. Each cyano group will be converted to a primary methyleneamine group. It has been found that when using the present process, the hydrogenation selectivity goes to the formation of primary amine product without any major interaction between the formed methyleneamine and the intermediate imine groups and especially substantially low intramolecular reaction and without the formation of N-methylated product.

The catalyst required to perform the present process is granular Raney cobalt. They catalyst should be charged into the reactor in an amount of from 5% to 20%, preferably 10% to 15% and most preferably about 10% by weight based on the weight of the polynitrile to be reacted in a particular run.

The catalyst is formed from an initial alloy which contains from 50 to 70 wt. percent aluminum, from 30 to 50 wt. percent cobalt, from 0 to 6 wt. percent chromium and from 0 to 6 wt. percent molybdenum. Chromium and/or molybdenum may also be provided by treating the surface of an already activated alloy with a salt of these materials to provide from 0 to 5 percent of these metals on the Raney cobalt surface. The most preferred catalyst is formed from alloys having small amounts (0.5 to 5 wt. percent) of chromium and/or molybdenum.

The catalyst can be prepared by contacting the starting alloy with an aqueous alkaline solution formed from an alkali or alkaline earth metal hydroxide, preferably sodium hydroxide. The alloy should be granular, that is have a particle size of from 20 to 50 μm (microns) mean diameter. The activation is carried out in known manners by contacting the starting alloy with dilute, normally from 1 to 10 wt. percent, preferably from 1 to 5 wt. percent, of an alkaline solution while maintaining a low temperature such as below about 50°C and preferably below 40°C. Generally, it is best to activate the alloy at from 20° to 40°C. Activation is readily monitored by the evolution of hydrogen and provides a suitable catalyst for use in the present process when from 20 to 40 percent of the aluminum is removed. The activated Raney cobalt catalyst is washed with water to free it from the alkaline solution and used immediately or stored under water or other inert medium or atmosphere.

The preferred catalyst for use in the present process is a chromium-promoted Raney cobalt catalyst commercially available under the designation Raney cobalt 2724 from the Davison Chemicals Division of W.R. Grace & Co., and is supplied with an average 30 $\mu$m (micron) particle size. As mentioned, this catalyst is relatively sensitive to oxygen and thus must be handled carefully to avoid surface oxidation. The catalyst particles are washed with deionized and deaerated water and then with deaerated inert solvent (preferably the same solvent used to prepare the feedstock solution) prior to use.

The Raney cobalt hydrogenation catalyst is quite sensitive to oxygen and thus oxygen should be excluded from each of the reactants and from the reaction vessels to the greatest extent possible. Nitrogen sparging of the reactant solutions and the storage of the same under nitrogen blankets is effective to exclude oxygen from the system.

Suitable hydrogenation reactor apparatuses preferred for carrying out the inventive process are known per se. A preferred type of fed-batch apparatus, the stirred autoclave, consists of a pressure vessel fitted with an agitator with cooling coils for heat removal and a means for introducing the feedstock solution during the pressurized reaction. Either an internal heating coil or an external heating jacket are employed to initially raise the reactants to a sufficient temperature. Also suitable is the so-called pump-loop reactor (e.g., manufactured by Buss, Pratteln, Switzerland).

Following completion of the hydrogenation reaction, the liquid contents of the vessel are separated from the solid catalyst. This separation can be accomplished by discharging the entire contents of the reactor vessel into a holding tank and then filtering the catalyst, or by allowing the catalyst to settle in the reactor and then removing the supernatant liquid via a dip tube in the reactor vessel. The catalyst has been found to retain its activity even after utility of numerous batch reactions when performed according to the present process. Therefore, utilization of fresh catalyst for each batch is not required when practising the present process.

The particulars of the present inventive process include:

Polynitrile feed solution: the polynitrile feedstock solution should be formed with a solvent which is inert to the reaction conditions found in the reaction zone and to the polynitrile reactant and polyamine product. Such solvents are discussed above. The concentration of polynitrile and the feedstock solution should be determined to maintain the proper rate of introduction of feedstock during the course of the batch reaction.

The Catalyst: The catalyst, as described above, should be added to the reaction zone under a nitrogen or other inert gas blanket. The catalyst is added in an amount of from 5 to 20 percent, preferably 10 to 15 percent and most preferably about 10 percent, by weight based on the weight of the polynitrile to be consumed during the fed-batch operation.

Ammonia: The presence of ammonia has been seen to improve the ratio of linear polyamine to the cyclic by-product in the overall reaction product. The molar ratio of ammonia to nitrile group of a given polynitrile reactant may range from 0 to above 4 with from 1 to 4 and especially about 2 being preferred.

Temperature: The reactor is heated to a temperature sufficient to start the hydrogenation reaction, for example to a temperature ranging from 90°C to 150°C, usually from 100°C to 130°C.

Pressure: The hydrogen pressure within the reaction zone should be maintained at a pressure of from 52730 to 2109210 kg/m$^2$ (750 to 3000 psig), preferably from 1054605 to 2109210 kg/m$^2$ (1500 to 3000 psig). The overall pressure maintained in the reaction zone should be sufficient to maintain the polynitrile and the solvent in a liquid state. The hydrogen pressure described above may be supplemented by partial pressure formed from an inert gas, such as nitrogen.

Polynitrile Feed: As stated above, the polynitrile should be introduced into the fed-batch reactor over the major time period of the batch reaction. The rate of introduction is to be governed by the molar concentration of the polynitrile in the feed solution and in the reaction zone as fully disclosed hereinabove. It has been unexpectedly found that by carrying out the batch process in a fed-batch manner, as described herein, one minimizes formation of cyclic by-product, minimizes the formation of condensation products, minimizes the formation of N-methylated amines of the corresponding desired product, produces a reaction product from which the desired polyamine is readily separated, and minimizes the deactivation of the catalyst and thus permits its reuse in subsequent runs.

The process can be conducted by sparging the reaction zone with an inert gas such as nitrogen and charging the zone with a heel of deaerated inert solvent of sufficient volume to make contact with the agitator and the temperature sensor. The catalyst is then charged to the reaction zone under a blanket of nitrogen. At this point, anhydrous ammonia may also be added. The reaction zone is then heated and charged with hydrogen pressure and the introduction of polynitrile solution into the reaction zone is commenced. The hydrogen pressure is generally maintained at a fixed level by continuous addition.

An extended fed-batch reaction can also be practised. In this case, once the reactor has been filled to its maximum operating level, continuation of the polynitrile feed can be maintained by the simultaneous withdrawal of filtered reaction product (free of catalyst) so as not to exceed the maximum operating level.

The following Examples illustrate the invention. All parts and percentages are by weight unless otherwise stated.

## Example 1

Into a one liter stainless steel autoclave previously flushed with nitrogen is charged 250 milliliters of dimethyl acetamide solvent along with 5.9 grams of solvent washed chromium promoted Raney® cobalt. Anhydrous ammonia (53.0 grams, 3.12 moles) was also added to the autoclave which was then heated to 90°C and pressurized to 1054605 kg/m$^2$ (1500 psig) with hydrogen. This temperature and pressure was maintained throughout the run.

A deaerated solution of iminodiacetonitrile (IDAN) in dimethylacetamide (125 grams, 23.7 wt. % IDAN) at room temperature was fed to the autoclave by a metering pump at a rate of 0.43 ml/minute over a period of 306 minutes. At the completion of the reaction the contents of the autoclave were removed and catalyst filtered. Analysis of the filtrate showed the yield of diethylene triamine (DETA) to be 43.3% of theoretical. The reaction parameter K, for this reaction was 19.7, lower than the desired range, which accounts for the low yield. The amount of N-methylated diethylene triamine impurity present amounted to 7.0% of the DETA product.

## Examples 2-6

The procedures of Example 1 were repeated except that conditions were varied to operate over a range of K values. The conditions and results are shown in Table 1 attached.

## Example 7

Chromium-promoted Raney® cobalt (12.5 g) was washed with deaerated dimethyl formamide (DMF) and added to a one liter autoclave, previously flushed with nitrogen, containing 250 ml. deaerated DMF. Anhydrous ammonia (34.0 g, 2 moles) was also added and the autoclave then was heated to 100°C and pressurized to 1054605 kg/m$^2$ (1500 psig) with hydrogen. This temperature and pressure was maintained throughout the run.

A deaerated solution of nitrilotriacetonitrile (NTAN) in DMF (195 ml, 25 wt. % NTAN) was fed to the autoclave by a metering pump at approximately the same rate at which it reacted (approx. 1 ml/min) over the course of 560 minutes. At the completion of the reaction, the contents of the autoclave were removed and the catalyst filtered. Analysis of the filtrate (592.0 g) showed the yield of TREN to be 79.6% of the theoretical value. The reaction parameter K is 68.4 for this run.

## Example 8

Following the procedure of Example 7, chromium promoted Raney® cobalt (4.9 g), anhydrous ammonia (34.0 g) and 250 ml. deaerated DMF were added to the oxygen-free autoclave which was then heated to 120°C and pressurized to 1054605 kg/m$^2$ (1500 psig) with hydrogen. A deaerated solution of NTAN in DMF (195 ml, 25 wt. % NTAN) was fed to the autoclave at a space velocity of 1.02. Analysis of the filtrate (495.3 g) from the reaction mixture showed a yield of 76.2% TREN. The reaction parameter K is 46.4 for this run.

## Example 9

The run described in Example 7 was repeated except that 195 ml. of a 5 wt. % NTAN in DMF was all added at the beginning of the run prior to pressurization to 2109210 kg/m$^2$ (3000 psig) with hydrogen and heating to 100°C. The yield of TREN dropped to 9% and the catalyst was found to have lost more than half its activity when it was reused.

A second run was conducted similar to that described in Example 7 except 150 gm deaerated DMAC was initially charged followed by 111 gm anhyd. ammonia and 500 gm DMAC solution having 25% IDAN (124 gm). All were added at the beginning of the run. The reactor was then pressurized and heated to 1054605 (1500 psig) H$_2$ and 125°C for 5 hours. The yield of DETA was 0.5%.

These runs show that when a typical batch process is carried out, very low yield and selectivity is obtained and the catalyst is poisoned.

6

## TABLE 1

| Example | Temperature °C | °K | Pressure Kg/M² (psig) | KPa (atm.) | Catalyst grams | Initial DMAC Charge ml | Initial Ammonia Charge grams | Total Initial Charge M x 10 | Feed Concentration Wt. % IDAN | IDAN Feed Solution grams | Feed Solution Flow M/hr x 10 | Run Time hour | K | DETA Yield mole % | N-Methyl DETA/DETA % |
|---|---|---|---|---|---|---|---|---|---|---|---|---|---|---|---|
| | (1) | (2) | (3) | (4) | (5) | (6) | (7) | (8) | (9) | (10) | (11) | (12) | (13) | (14) | (15) |
| 1 | 90 | 363 | 703070 (1000) | 6991 (69) | 5.9 | 250 | 53 | 3.06 | 23.7 | 125 | 2.58 | 5.1 | 19.7 | 43.3 | 7.0 |
| 2 | 90 | 363 | 703070 (1000) | 6991 (69) | 5.9 | 250 | 56 | 3.09 | 23.7 | 250 | 4.98 | 5.3 | 10.2 | 15.9 | N.A. |
| 3 | 90 | 363 | 703070 (1000) | 6991 (69) | 3.3 | 250 | 31 | 2.83 | 23.7 | 139 | 4.98 | 2.9 | 5.7 | 34.8 | N.A. |
| 4 | 125 | 398 | 1054605 (1500) | 10436 (103) | 5.9 | 250 | 26 | 2.78 | 23.7 | 125 | 2.58 | 5.1 | 78.0 | 82.7 | 1.7 |
| 5 | 125 | 398 | 1054605 (1500) | 10436 (103) | 5.9 | 250 | 26 | 2.78 | 23.7 | 125 | 3.96 | 3.3 | 50.8 | 87.0 | N.A. |
| 6 | 125 | 398 | 1054605 (1500) | 10436 (103) | 27.9 | 150 | 125 | 2.81 | 23.7 | 494 | 18.0 | 2.9 | 52.7 | 80.0 | 1.6 |

N.A. = not available

## Claims

1. A process for the batchwise preparation of non-cyclic, aliphatic polyamines, comprising:

a) providing a feedstock solution wherein a non-cyclic aliphatic polynitrile is gradually introduced into a fed-batch reactor vessel over substantially the entire time of the reaction at a rate no greater than the maximum rate at which the polynitrile contained within the reactor vessel reacts with hydrogen;

b) adding said feedstock solution to said fed-batch reactor vessel containing a Raney cobalt catalyst in a substantially oxygen-free atmosphere; and

c) hydrogenating the polynitrile by contacting said polynitrile with hydrogen in the reactor vessel.

2. A process according to Claim 1 wherein the polynitrile is nitrilotriacetonitrile and the polyamine product is tris(2-aminoethyl) amine.

3. A process according to Claim 1 wherein the polynitrile is iminodiacetonitrile and the polyamine product is diethylenetriamine.

4. A process according to Claim 1 wherein the polynitrile is ethylenediaminetetraacetonitrile and the polyamine product is tetrakis(2-aminoethyl)ethylenediamine.

5. A process according to any one of the preceding Claims further comprising adding anhydrous ammonia to the reactor vessel in an amount of 1 to 4 moles of ammonia per mole of nitrile group contained in the polynitrile.

6. A process according to any one of the preceding Claims wherein the catalyst is chromium-promoted Raney cobalt.

7. A process according to any one of the preceding Claims wherein said feedstock solution comprises a substantially saturated solution of polynitrile in inert solvent.

8. A process according to any one of the preceding Claims wherein said catalyst is present in an amount of from 5 to 20 percent by weight based on the weight of the polynitrile to be consumed during the fed-batch reaction.

9. A process according to any one of the preceding Claims wherein the polynitrile is introduced into the reactor vessel at a rate such that the molar concentration of polynitrile within the reactor is equal to or less than three mole percent of the polynitrile within the feed solution.

10. A process according to any one of the preceding Claims wherein the polynitrile is introduced into the reactor vessel at a space velocity of from 0.1 to 1.3.

11. A process according to any one of the preceding Claims wherein the volumetric feed rate is such that it provides a reaction parameter K, having a value of 30 or greater, which is defined by the equation:

$$K = \frac{163 \, WP}{nV_f} \cdot e^{-\frac{4026}{T+273}}$$

wherein

W = amount of catalyst in the reaction zone in kilograms;

P = hydrogen pressure in the reaction zone in atmospheres;

$V_f$ = volumetric feed rate of the polynitrile feed solution into the reactor in cubic meters per hour;

n = number of nitrile groups per mole of feed; and

T = reaction zone temperature in °C.

12. A process according to any one of the preceding claims wherein the process is conducted by an extended fed-batch reaction.

**Patentansprüche**

1. Verfahren zur chargenweisen Herstellung von nicht-cyclischen, aliphatischen Polyaminen, bei dem:
   a) eine Einsatzmateriallösung geliefert wird, wobei ein nicht-cyclisches, aliphatisches Polynitril graduell in ein Zufuhr-Chargen-Reaktorgefäß über im wesentlichen den gesamten Reaktionszeitraum mit einer Rate von nicht größer als der maximalen Rat zugeführt wird, mit der das in dem Reaktorgefäß enthaltene Polynitril mit Wasserstoff reagiert,
   b) diese Einsatzmateriallösung in das Zufuhr-Chargen-Reaktorgefäß gegeben wird, das einen Raney-Kobalt-Katalysator in einer im wesentlichen sauerstofffreien Atmosphäre enthält, und
   c) das Polynitril hydriert wird, indem das Polynitril mit dem Wasserstoff in dem Reaktorgefäß kontaktiert wird.

2. Verfahren nach Anspruch 1, bei dem das Polynitril Nitrilotriacetonitril ist und das Polyaminprodukt Tris-(2-aminoethyl)amin ist.

3. Verfahren nach Anspruch 1, bei dem das Polynitril Iminodiacetonitril ist und das Polyaminprodukt Diethylentriamin ist.

4. Verfahren nach Anspruch 1, bei dem das Polynitril Ethylendiamintetra-acetonitril ist und das Polyaminprodukt Tetrakis(2-aminoethyl)ethylendiamin ist.

5. Verfahren nach einem der vorhergehenden Ansprüche, bei dem dem Reaktorgefäß ferner wasserfreies Ammoniak in einer Menge von 1 bis 4 Mole Ammoniak pro Mol der in dem Polynitril enthaltenden Nitrilgruppe zugesetzt wird.

6. Verfahren nach einem der vorhergehenden Ansprüche, bei dem der Katalysator Chrom-aktiviertes Raney-Kobalt ist.

7. Verfahren nach einem der vorhergehenden Ansprüche, bei dem die Einsatzmateriallösung eine im wesentlichen gesättigte Lösung von Poly-nitril in inertem Lösungsmittel umfaßt.

8. Verfahren nach einem der vorhergehenden Ansprüche, bei dem der Katalysator in einer Menge von 5 bis 20 Gew.-%, bezogen auf das Gewicht des während der Zufuhr-Chargen-Reaktion zu verbrauchenden Polynitrils, vorhanden ist.

9. Verfahren nach einem der vorhergehenden Ansprüche, bei dem das Polynitril mit einer solchen Rate in das Reaktorgefäß eingeführt wird, das die molare Konzentration an Polynitril in dem Reaktor gleich wie oder kleiner ist als 3 Mol-% des Polynitrils in der Einsatzmateriallösung.

10. Verfahren nach einem der vorhergehenden Ansprüche, bei dem das Polynitril mit einem Durchsatz von 0,1 bis 1,3 in das Reaktorgefäß eingeführt wird.

11. Verfahren nach einem der vorhergehenden Ansprüche, bei dem die volumetrische Zufuhrrate so ist, daß sie einen Reaktionsparameter K mit einem Wert von 30 oder größer liefert, der durch die Gleichung:

$$K = \frac{163 \, \text{WP} \cdot e^{\frac{-4026}{T+273}}}{nV_f}$$

definiert ist,
wobei

W = der Menge an Katalysator in der Reaktionszone in kg ist,
P = dem Wasserstoffdruck in der Reaktionszone in Atmosphären ist,
$V_f$ = der volumetrischen Zufuhrrate der Polynitrileinsatzmateriallösung in den Reaktor in $m^3$/h ist,
n = der Zahl von Nitrolgruppen pro Mol Einsatzmaterial ist und
T = der Reaktionszonentemperatur in °C ist.

12. Verfahren nach einem der vorhergehenden Ansprüche, bei dem das Verfahren mittels einer ausgedehnten Zufuhr-Chargen-Reaktion durchgeführt wird.

**Revendications**

1. Procédé pour la préparation discontinue de polyamides aliphatiques non cycliques, consistant à :
   a) prévoir une solution de charge d'alimentation où un polynitrile aliphatique non cyclique est graduellement introduit dans un récipient de réacteur à alimentation discontinue, sensiblement sur tout le temps de la réaction, à une allure qui n'est pas plus importante que l'allure maximale à laquelle le polynitrile contenu dans le récipient du réacteur réagit avec l'hydrogène ;
   b) ajouter ladite solution de la charge d'alimentation audit récipient du réacteur à alimentation discontinue contenant un catalyseur de cobalt de Raney dans une atmosphère sensiblement sans oxygène ; et
   c) hydrogéner le polynitrile par contact dudit polynitrile avec de l'hydrogène dans le récipient du réacteur.

2. Procédé selon la revendication 1, où le polynitrile est du nitrilotriacétonitrile et la polyamine produite est la tris(2-aminoéthyl)amine.

3. Procédé selon la revendication 1, où le polynitrile est l'iminodiacétonitrile et la polyamine produite est la diéthylènetriamine.

4. Procédé selon la revendication 1, où le polynitrile est l'éthylènediaminetétraacétonitrile et la polyamine produite est la tétrakis(2-aminoéthyl)éthylènediamine.

5. Procédé selon l'une quelconque des revendications précédentes consistant de plus à ajouter de l'ammoniaque anhydre au récipient du réacteur en une quantité de 1 à 4 moles d'ammoniaque par mole de groupe nitrile contenu dans le polynitrile.

6. Procédé selon l'une quelconque des revendications précédentes, où le catalyseur est du cobalt de Raney promu par du chrome.

7. Procédé selon l'une quelconque des revendications précédentes, où ladite solution de la charge d'alimentation comprend une solution sensiblement saturée de polynitrile dans un solvant inerte.

8. Procédé selon l'une quelconque des revendications précédentes, où ledit catalyseur est présent en une quantité de 5 à 20 pour cent en poids, en se basant sur le poids du polynitrile à consommer pendant la réaction à alimentation discontinue.

9. Procédé selon l'une quelconque des revendications précédentes, où le polynitrile est introduit dans le récipient du réacteur à une allure telle que la concentration molaire du polynitrile dans le réacteur soit égale à ou plus faible que trois pour cent en moles du polynitrile dans la solution d'alimentation.

10. Procédé selon l'une quelconque des revendications précédentes, où le polynitrile est introduit dans le récipient du réacteur à une vitesse spatiale comprise entre 0,1 et 1,3.

11. Procédé selon l'une quelconque des revendications précédentes, où le taux d'alimentation volumétrique est tel que cela forme un paramètre de réaction K, ayant une valeur de 30 ou plus, qui est défini par l'équation :

$$K = \frac{163 \; WP \cdot e^{\frac{-4026}{T+273}}}{nV_f}$$

où

W = quantité du catalyseur dans la zone réactionnelle en kilogrammes ;

P = pression d'hydrogène dans la zone réactionnelle en atmosphères ;

$V_f$ = taux d'alimentation volumétrique de la solution d'alimentation du polynitrile dans le réacteur en mètres cubes par heure ;

n = nombre de groupes nitriles par mole de l'alimentation ; et

T = température dans la zone de réaction en °C.

12. Procédé selon l'une quelconque des revendications précédentes, où le procédé est entrepris par une réaction à alimentation discontinue étendue.